# EUROPÄISCHE PATENTSCHRIFT

(11) **EP 3 334 474 B1**
(45) Veröffentlichungstag und Bekanntmachung des Hinweises auf die Patenterteilung: **25.09.2024**
(21) Anmeldenummer: 16757556.2
(22) Anmeldetag: 09.08.2016
(51) Int. Cl.: A61M 1/14, A61M 1/28, G06K 13/08

(54) **BLUTBEHANDLUNGSGERÄT**
BLOOD TREATMENT APPARATUS
APPAREIL DE TRAITEMENT DE SANG

(30) Priorität: 11.08.2015 DE 102015010430
(43) Veröffentlichungstag der Anmeldung: 20.06.2018
(73) Patentinhaber: Fresenius Medical Care Deutschland GmbH, 61352 Bad Homburg (DE)
(72) Erfinder: ZEYHER, Peter, 64289 Darmstadt (DE); FRITSCH, Kim, 65468 Trebur (DE)
(74) Vertreter: Herrmann, Uwe
(86) Internationale Anmeldenummer: PCT/EP2016/001363
(87) Internationale Veröffentlichungsnummer: WO 2017/025185

(56) Entgegenhaltungen:
- EP-A2- 1 574 178
- WO-A1-2013/014706
- DE-A1- 102011 105 916
- US-A- 5 225 653
- US-A1- 2003 088 203
- US-A1- 2012 307 451
- US-A1- 2016 162 712

## Beschreibung

Die vorliegende Erfindung betrifft ein Blutbehandlungsgerät mit wenigstens einer Lese- und/oder Schreibvorrichtung, die ausgebildet ist, Informationen von einer Karte zu lesen und/oder Informationen auf eine Karte zu schreiben, und mit wenigstens einer Aufnahme, in die die Karte einführbar und aus der die Karte entnehmbar ist, wobei die Aufnahme derart angeordnet ist, dass im in der Aufnahme eingeführten Zustand der Karte Informationen mittels der Lese- und/oder Schreibvorrichtung von der Karte gelesen und/oder auf die Karte geschrieben werden können.

Aus dem Stand der Technik sind Blutbehandlungsgeräte insbesondere in Form von Peritonealdialysegeräten bekannt, bei denen die Lösungsbeutel, die das Dialysat enthalten, auf oder über dem eigentlichen Gerät bzw. Gerätegehäuse angeordnet sind.

Dabei besteht das Risiko, dass aus den Beuteln austretende Flüssigkeit auf das Gerätegehäuse und insbesondere auf eine in der Aufnahme befindliche Chipkarte, wie beispielsweise eine Patientenkarte, tropft und sodann in das Gerät eindringt. Die eindringende Flüssigkeit kann Schäden an der Elektronik, wie beispielsweise an der Lese- und/oder Schreibvorrichtung im Gerät verursachen bzw. diese verschmutzen, sodass die Lesbarkeit bzw. die Beschreibbarkeit der Karte entsprechend beeinträchtigt ist.

Die DE102011105916A1 offenbart eine Dialysemaschine mit einem Kartenleser, über welchen eine Patientenkarte eingelesen werden kann. US5,225,653 offenbart einen Chipkartenempfänger mit schräg verlaufender Führung für dünne Chipkarten. Aus der WO2013/014706A1 ist ein mit einer Chipkarte bedienbarer Geldautomat bekannt. Die US2012/307451A1 offenbart ein Modul zum Auswerfen einer Komponente aus einem elektronischen Gerät.

Der vorliegenden Erfindung liegt die Aufgabe zu Grunde, ein Blutbehandlungsgeräte der eingangs genannten Art dahingehend weiterzubilden, dass die Wahrscheinlichkeit für das Eindringen von Flüssigkeit in die Aufnahme gegenüber bekannten Geräten verringert ist.

Diese Aufgabe wird durch ein Blutbehandlungsgerät mit den Merkmalen des Anspruchs 1 gelöst.

Demnach ist vorgesehen, dass die Aufnahme nicht horizontal ausgeführt ist, sondern gegenüber der Horizontalen in Einführrichtung der Karte abschnittsweise oder insgesamt schräg nach oben ausgerichtet ist.

Durch diese Neigung der Aufnahme wird mit vergleichsweise einfachen Mitteln erreicht, dass das Eindringen von Flüssigkeit in die Aufnahme vermieden wird. Sollte Flüssigkeit auf die eingesteckte Chipkarte auftreffen oder in den Bereich der Einführöffnung der Aufnahme gelangen, läuft diese nach unten bzw. nach außen ab und nicht in die Aufnahme hinein.

Derartige Karten sind üblicherweise Patientenkarten, auf denen Daten des Patienten bzw. der Behandlung am Blutbehandlungsgerät aufgezeichnet sind.

Bei der Karte handelt es sich erfindungsgemäß um eine Chipkarte, d.h. eine Karte mit integriertem Schaltkreis, der z.B. einen Speicher oder auch einen Mikroprozessor aufweist.

In einer bevorzugten Ausgestaltung der Erfindung handelt es sich um eine Kunststoffkarte, wie sie beispielsweise auch bei EC-Karten bekannt ist.

Die Lese- und/oder Schreibvorrichtung ist ausgebildet, um Informationen von der Chipkarte zu lesen und/oder Informationen auf die Chipkarte oder sonstige Karte zu schreiben. Diese Informationen können beispielsweise die gerade durchgeführte oder gerade laufende Blutbehandlung betreffen.

Die Aufnahme weist wenigstens eine Einführöffnung, vorzugsweise einen Schlitz auf, wobei sich die Lese- und/oder Schreibvorrichtung in einem Bereich befindet, der von der Einführöffnung beabstandet ist.

Vorzugsweise befindet sich die Lese- und/oder Schreibvorrichtung in dem von der Einführöffnung beabstandeten Endbereich der Aufnahme.

Dadurch ist besonders zuverlässig sichergestellt, dass auf die Chipkarte tropfende Flüssigkeit nicht in dem Bereich des Kartenchips bzw. in den Bereich der Lese- und/oder Schreibvorrichtung gelangt.

In bevorzugter Ausgestaltung der Erfindung handelt es sich bei dem Blutbehandlungsgerät um ein Dialysegerät.

Besonders bevorzugt ist es, wenn es sich bei dem Blutbehandlungsgerät um ein Peritonealdialysegerät handelt.

Bei einem Peritonealdialysegerät ist üblicherweise ein Gerätegehäuse mit der gesamten Gerätesteuerung bzw. -regelung vorhanden, wobei vorzugsweise vorgesehen ist, dass sich die Aufnahme für die Karte in diesem Gehäuse befindet. Oberhalb von diesem Gehäuse befinden sich üblicherweise Aufnahmen bzw. Einhängevorrichtungen für Lösungsbeutel, die mit Dialyselösung gefüllt sind.

Gelangt Flüssigkeit aus diesen Beuteln in den Bereich der Aufnahme, wird durch die schräge Anordnung der Aufnahme verhindert, dass die Flüssigkeit in die Aufnahme hineinläuft. Vielmehr wird erreicht, dass die Flüssigkeit nach außen hin abläuft bzw. abtropft.

Vorzugsweise weist die Aufnahme wenigstens eine Einführöffnung, vorzugsweise wenigstens einen Einführschlitz für die Karte auf, wobei im Bereich der Einführöffnungen keine gesonderten Mittel zur Vermeidung des Eindringens von Flüssigkeit in die Einführöffnung vorhanden sind. Dies ermöglicht einen besonders einfachen Aufbau, bei dem beispielsweise auf Vorsprünge etc., die die Flüssigkeit von der Aufnahme wegführen oder abhalten verzichtet werden kann. Das Eindringen von Flüssigkeit in die Aufnahme hinein wird vorzugsweise ausschließlich durch deren schräge Ausführung verhindert.

Vorzugsweise ist auch keine Abdeckung für die Einführöffnung vorhanden, da - wie ausgeführt - das Eindringen von Flüssigkeit in die Aufnahme hinein durch deren schräge Anordnung verhindert wird.

Der Winkel zwischen der Aufnahme und der Horizontalen liegt vorzugsweise im Bereich von < 45°, vorzugsweise im Bereich < 20°. Ein besonders vorteilhafter Winkel liegt bei 10° bis 20° und beispielsweise bei 15°.

Die Aufnahme verläuft erfindungsgemäß über ihre gesamte sich in Einführrichtung der Karte erstreckende Länge schräg nach oben.

Bevorzugt ist es, wenn die Aufnahme insgesamt schräg ausgeführt ist und nur eine einzige Steigung aufweist. Grundsätzlich sind jedoch auch abweichende Ausführungsformen umfasst. Ändert sich die Neigung der Aufnahme entlang der Einführrichtung, erfordert dies eine entsprechend flexible ausgeführte Karte.

Die Aufnahme ist mehrteilig ausgeführt, wobei i zwischen den Teilen der Aufnahme ein Spalt vorhanden sein kann.

Die Form der Aufnahme ist beliebig. Erfindungsgemäß handelt es sich um einen Hohlraum, der entsprechend der Kontur der Karte im Wesentlichen rechteckig ausgebildet ist.

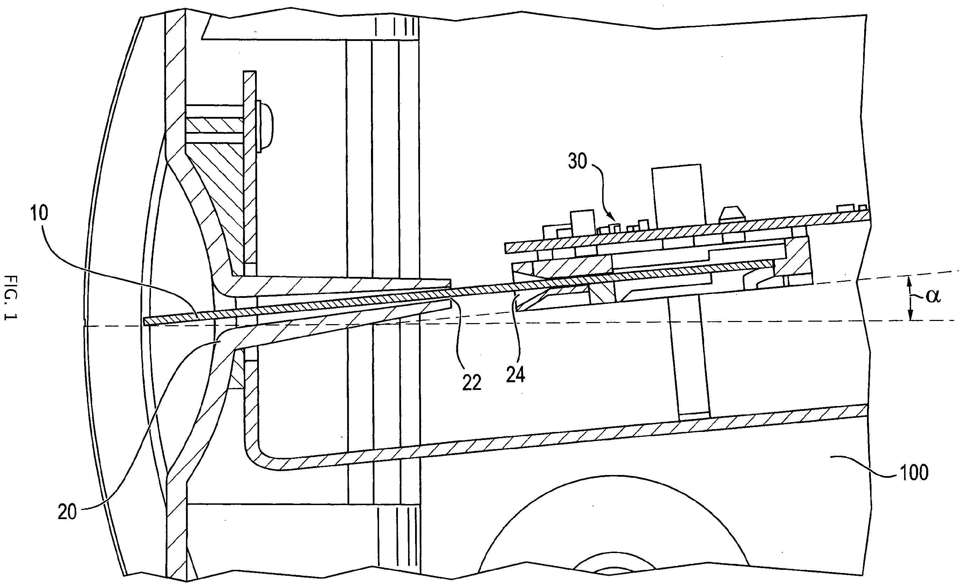

Weitere Einzelheiten und Vorteile der Erfindung werden anhand eines des in der Zeichnung dargestellten Ausführungsbeispiels näher erläutert.

Die einzige Zeichnung zeigt einen Schnitt durch das Gehäuse eines Peritonealdialysegerätes im Bereich der Kartenaufnahme.

Das Bezugszeichen 100 kennzeichnet das Chassis bzw. das Gehäuse eines Peritonealdialysegerätes, in dem sich die Elektronik zur Durchführung der Blutbehandlung befindet. Oberhalb des Gehäuses 100 befinden sich nicht dargestellte Einhängemöglichkeiten für Lösungsbeutel, die das für die Behandlung benötigte Dialysat enthalten.

Mit dem Bezugszeichen 10 ist eine Patientenkarte gekennzeichnet, bei der es sich um eine Chipkarte handelt, die in der Aufnahme 22, 24 aufgenommen ist. Die Aufnahme 22, 24 wird durch einen Hohlraum gebildet, der in seinen Abmessungen in etwa den Außenabmessungen der Karte 10 entspricht.

Das Bezugszeichen 20 kennzeichnet den Einführschlitz für die Karte 10.

Wie dies aus der Figur hervorgeht, ist erfindungsgemäß die Aufnahme 22, 24 mehrteilig ausgeführt, wobei die mehreren Teile 22, 24 voneinander beabstandet sind. Die Karte 10 durchläuft in Einschubrichtung zunächst den Teil 22 der Aufnahme und sodann den Teil 24 der Aufnahme, wobei dieser Teil eine trichterförmige Einführöffnung hat, um sicherzustellen, dass die Karte beim Einführen zuverlässig auch in den Teil 24 der Aufnahme gelangt.

Das Bezugszeichen 30 kennzeichnet eine Lese- und/oder Schreibvorrichtung, die ausgebildet ist, um die Daten der Patientenkarte 10 bzw. von deren Chip auszulesen und/oder darauf Informationen zu schreiben.

Wie dies aus der Abbildung hervorgeht, ist gemäß dem Ausführungsbeispiel die Aufnahme so ausgebildet, dass sie eine Tiefe hat, die unter der Länge der Chipkarte 10 in Einführrichtung liegt, sodass die Chipkarte im eingeführten Zustand außen über den Einführschlitz 20 übersteht.

Um nun zu vermeiden, dass insbesondere auf diesen überstehenden Teil der Karte 10 tropfende Flüssigkeit in die Aufnahme 22, 24 und insbesondere in den Bereich der Aufnahme 24 läuft, in dem die Lese- und/oder Schreibvorrichtung angeordnet ist, ist die Aufnahme 22, 24 insgesamt um den Winkel α gegenüber der Horizontalen geneigt. In Einführrichtung der Karte 10 ist die Aufnahme 22, 24 nach oben geneigt, sodass die auf den überstehenden Teil der Karte 10 tropfende Flüssigkeit nicht in die Aufnahme hineinläuft, sondern von der Karte außerhalb der Aufnahme abtropft.

Bei dem Winkel α handelt es sich beispielsweise um einen Winkel von 15°, wobei dieser Winkel nur exemplarischer Natur ist.

Im Bereich des Einführschlitzes 20 befindet sich weder ein Vorsprung noch eine Klappe, der herabtropfende Flüssigkeit abhält. Dies ist auch nicht notwendig, da die Neigung der Aufnahme das Hineinlaufen der Flüssigkeit in die Aufnahme 22, 24 verhindert. Die vergleichsweise geringe Neigung der Aufnahme gegenüber der Horizontalen erlaubt eine im Wesentlichen horizontale Einführrichtung der Karte, die dann entsprechend der Neigung der Aufnahme ausgerichtet wird, d.h. schräg nach oben weist bzw. eingeführt wird.

## Patentansprüche

1. Blutbehandlungsgerät mit wenigstens einer Lese- und/oder Schreibvorrichtung, die ausgebildet ist, Informationen von einer Chipkarte zu lesen und/oder Informationen auf eine Chipkarte zu schreiben und mit wenigstens einer Aufnahme, in die die Chipkarte einführbar und aus der die Chipkarte entnehmbar ist, wobei die Aufnahme derart angeordnet ist, dass im in der Aufnahme eingeführten Zustand der Chipkarte Informationen mittels der Lese- und/oder Schreibvorrichtung von der Chipkarte gelesen und/oder auf die Chipkarte geschrieben werden können,
**dadurch gekennzeichnet,**
**dass** die Aufnahme nicht horizontal ausgeführt ist, sondern gegenüber der Horizontalen über ihre gesamte sich in Einführrichtung der Chipkarte erstreckende Länge schräg nach oben verläuft, dass die Aufnahme durch einen Hohlraum gebildet wird, der in seinen Abmessungen den Außenabmessungen der Chipkarte entspricht und dass die Aufnahme mehrteilig ausgeführt ist und einen ersten und einen zweiten Teil umfasst, wobei der erste Teil und der zweite Teil voneinander beabstandet sind und wobei die Chipkarte in Einschubrichtung zuerst den ersten Teil und sodann den zweiten Teil durchläuft und wobei der erste Teil eine trichterförmige Einführöffnung hat, um sicherzustellen, dass die Chipkarte beim Einführen zuverlässig auch in den zweiten Teil der Aufnahme gelangt.

2. Blutbehandlungsgerät nach Anspruch 1, **dadurch gekennzeichnet, dass** die Lese- und/oder Schreibvorrichtung ausgebildet ist, Informationen von der Chipkarte zu lesen und/oder Informationen auf die Chipkarte zu schreiben.

3. Blutbehandlungsgerät nach Anspruch 1 oder 2, **dadurch gekennzeichnet, dass** die Aufnahme wenigstens eine Einführöffnung für die Chipkarte aufweist und dass sich die Lese- und/oder Schreibvorrichtung in dem von der Einführöffnung beabstandeten Endbereich der Aufnahme befindet.

4. Blutbehandlungsgerät nach einem der vorhergehenden Ansprüche, **dadurch gekennzeichnet, dass** es sich bei dem Blutbehandlungsgerät um ein Dialysegerät handelt.

5. Blutbehandlungsgerät nach einem der vorhergehenden Ansprüche, **dadurch gekennzeichnet, dass** es sich bei dem Blutbehandlungsgerät um ein Peritonealdialysegerät handelt.

6. Blutbehandlungsgerät nach einem der vorhergehenden Ansprüche, **dadurch gekennzeichnet**, sich die Aufnahme in einem Gehäuse befindet und dass sich oberhalb des Gehäuses ein oder mehrere Lösungsbeutel befinden oder dass ein oder mehrere Aufnahmen für Lösungsbeutel oberhalb des Gehäuses angeordnet sind.

7. Blutbehandlungsgerät nach einem der vorhergehenden Ansprüche, **dadurch gekennzeichnet, dass** die Aufnahme wenigstens eine Einführöffnung für die Karte aufweist und dass im Bereich der Einführöffnung keine Mittel zur Vermeidung des Eindringens von Flüssigkeit in die Einführöffnung vorhanden sind.

8. Blutbehandlungsgerät nach einem der vorhergehenden Ansprüche, **dadurch gekennzeichnet, dass** die Aufnahme wenigstens eine Einführöffnung für die Karte aufweist und dass keine Abdeckung für die Einführöffnung vorhanden ist.

9. Blutbehandlungsgerät nach einem der vorhergehenden Ansprüche, **dadurch gekennzeichnet, dass** der Winkel zwischen der Aufnahme und der Horizontalen im Bereich von < 45°, vorzugsweise im Bereich < 30° und besonders bevorzugt im Bereich von < 20° liegt.

## Claims

1. Blood treatment device having at least one reading and/or writing apparatus which is configured to read information from a chip card and/or to write information to a chip card, and having at least one receiver into which the chip card can be inserted and from which the chip card can be removed, wherein the receiver is arranged such that information can be read from the chip card and/or information can be written to the chip card by means of the reading and/or writing apparatus in the state of the chip card being inserted into the receiver,
**characterized in that**
the receiver is not horizontal, but rather extends slanted upwardly with respect to the horizontal in the entire insertion direction of the chip card, that the receiver is formed by a void, the outer dimensions of which correspond to the outer dimensions of the chip card and that the receiver is configured in multiple parts and comprises a first and a second part, wherein the first part and the second part are spaced from one another and wherein the chip card first passes through the first part and then through the second part in the insertion direction in order to ensure that the chipcard reliably also enters the second part of the receiver when being inserted.

2. Blood treatment device in accordance with claim 1, **characterized in that** the reading and/or writing apparatus is configured to read information from the chip card and/or to write information to the chip card.

3. Blood treatment device in accordance with claim 1 or claim 2, **characterized in that** the receiver has at least one insertion opening for the card; and **in that** the reading and/or writing apparatus is located in the end region of the receiver spaced apart from the insertion opening.

4. Blood treatment device in accordance with any one of the preceding claims, **characterized in that** the blood treatment device is a dialysis machine.

5. Blood treatment device in accordance with any one of the preceding claims, **characterized in that** the blood treatment device is a peritoneal dialysis machine.

6. Blood treatment device in accordance with any one of the preceding claims, **characterized in that** the receiver is located in a housing; and **in that** one or more solution bags are located above the housing; or **in that** one or more receivers for solution bags are arranged above the housing.

7. Blood treatment device in accordance with any one of the preceding claims, **characterized in that** the receiver has at least one insertion opening for the card; and **in that** no means for avoiding the penetration of liquid into the insertion opening are present in the region of the insertion opening.

8. Blood treatment device in accordance with any one of the preceding claims, **characterized in that** the receiver has at least one insertion opening for the card; and **in that** no cover for the insertion opening is present.

9. Blood treatment device in accordance with any one of the preceding claims, **characterized in that** the angle between the receiver and the horizontal is in the range of < 45°, preferably in the range of < 30°, and particularly preferably in the range of < 20°.

## Revendications

1. Appareil de traitement de sang avec au moins un dispositif de lecture et/ou d'écriture qui est configuré pour lire des informations d'une carte à puce et/ou pour écrire des informations sur une carte à puce et avec au moins un logement dans lequel la carte à puce peut être introduite et duquel la carte à puce peut être retirée, le logement étant agencé de telle sorte que, lorsque la carte à puce est introduite dans le logement, des informations peuvent être lues de la carte à puce et/ou écrites sur la carte à puce au moyen du dispositif de lecture et/ou d'écriture,
**caractérisé en ce que**
le logement n'est pas réalisé horizontalement, mais s'étend obliquement vers le haut par rapport à l'horizontale sur toute sa longueur s'étendant dans la direction d'introduction de la carte à puce, **en ce que** le logement est formé par une cavité dont les dimensions correspondent aux dimensions extérieures de la carte à puce et **en ce que** le logement est réalisé en plusieurs parties et comprend une première et une deuxième partie, la première partie et la deuxième partie étant espacées l'une de l'autre et la carte à puce traversant d'abord la première partie puis la deuxième partie dans la direction d'insertion, et la première partie comportant une ouverture d'introduction en forme d'entonnoir afin de garantir que la carte à puce passe de manière fiable dans la deuxième partie du logement lors de l'introduction.

2. Appareil de traitement de sang selon la revendication 1, **caractérisé en ce que** le dispositif de lecture et/ou d'écriture est configuré pour lire des informations de la carte à puce et/ou pour écrire des informations sur la carte à puce.

3. Appareil de traitement de sang selon la revendication 1 ou 2, **caractérisé en ce que** le logement présente au moins une ouverture d'introduction pour la carte à puce et **en ce que** le dispositif de lecture et/ou d'écriture se trouve dans la zone d'extrémité du logement espacée de l'ouverture d'introduction.

4. Appareil de traitement de sang selon l'une quelconque des revendications précédentes, **caractérisé en ce que** l'appareil de traitement de sang consiste en un appareil de dialyse.

5. Appareil de traitement de sang selon l'une quelconque des revendications précédentes, **caractérisé en ce que** l'appareil de traitement de sang consiste en un appareil de dialyse péritonéale.

6. Appareil de traitement de sang selon l'une quelconque des revendications précédentes, **caractérisé en ce que** le logement se trouve dans un boîtier et **en ce qu'**une ou plusieurs poches de solution se trouvent au-dessus du boîtier ou **en ce qu'**un ou plusieurs logements pour poches de solution sont agencés au-dessus du boîtier.

7. Appareil de traitement de sang selon l'une quelconque des revendications précédentes, **caractérisé en ce que** le logement présente au moins une ouverture d'introduction de la carte et **en ce qu'**il n'existe pas, au niveau de l'ouverture d'introduction, de moyens permettant d'éviter que le liquide ne pénètre dans l'ouverture d'introduction.

8. Appareil de traitement de sang selon l'une quelconque des revendications précédentes, **caractérisé en ce que** le logement présente au moins une ouverture d'introduction pour la carte et **en ce qu'**aucun couvercle pour l'ouverture d'introduction n'est présent.

9. Appareil de traitement de sang selon l'une quelconque des revendications précédentes, **caractérisé en ce que** l'angle entre le logement et l'horizontale se situe dans la plage de < 45°, de préférence dans la plage de < 30° et de manière particulièrement préférée dans la plage de < 20°.
